(19)

Europäisches Patentamt

European Patent Office

Office européen des brevets

(11) **EP 1 603 506 B1**

(12) **EUROPEAN PATENT SPECIFICATION**

(45) Date of publication and mention
of the grant of the patent:
**15.05.2013 Bulletin 2013/20**

(51) Int Cl.:
***A61F 13/15*** *(2006.01)*

(21) Application number: **04720306.2**

(86) International application number:
**PCT/SE2004/000364**

(22) Date of filing: **12.03.2004**

(87) International publication number:
**WO 2004/098474 (18.11.2004 Gazette 2004/47)**

(54) **ABSORBENT ARTICLE WITH IMPROVED SURFACE MATERIAL**

SAUGFÄHIGER ARTIKEL MIT VERBESSERTEM OBERFLÄCHENMATERIAL

ARTICLE ABSORBANT A MATIERE DE SURFACE AMELIOREE

(84) Designated Contracting States:
**AT BE BG CH CY CZ DE DK EE ES FI FR GB GR HU IE IT LI LU MC NL PL PT RO SE SI SK TR**

(30) Priority: **14.03.2003 SE 0300694**

(43) Date of publication of application:
**14.12.2005 Bulletin 2005/50**

(73) Proprietor: **SCA Hygiene Products AB**
**405 03 Göteborg (SE)**

(72) Inventors:
• **NIHLSTRAND, Anna**
**S-431 35 Mölndal (SE)**

• **MOBERG Barbro**
**S-421 43 Västra Frölunda (SE)**
• **STORM, Anna-Karin**
**S-654 67 Karlstad (SE)**
• **BAGGER-SJÖBACK, Anna**
**S-414 63 Göteborg (SE)**

(74) Representative: **Romare, Laila Anette et al**
**Zacco Sweden AB**
**Torggatan 8**
**Box 142**
**401 22 Göteborg (SE)**

(56) References cited:
**EP-A2- 0 312 118     WO-A1-98/51250**
**US-A- 5 728 081     US-A- 5 968 855**

**Description**

TECHNICAL FIELD

The invention relates to an absorbent article comprising an absorbent body, a liquid-permeable covering layer arranged over a first surface on the absorbent body, and a liquid-permeable liquid-transfer layer arranged between the absorbent body and the liquid-permeable covering layer.

BACKGROUND ART

Disposable absorbent articles, such as diapers, incontinence pads, bed protectors, sanitary towels etc., which are intended to receive and absorb bodily discharges such as urine, menstrual blood and motions have been well known for a long time. Absorbent articles of this kind usually comprise a liquid-permeable covering layer and a liquid-impermeable covering layer, with an absorbent body enclosed between the covering layers. Especially in the case of urine absorption, it is essential in this connection that the absorbent article and in particular the liquid-permeable covering layer is capable of receiving and rapidly admitting liquid. It is also important for the surface of the article to be kept as dry as possible even after wetting and for liquid which has passed into the article to remain there and not leak back out towards the body of the user. This phenomenon is usually referred to as rewetting and is highly undesirable.

The requirements for rapid admission of liquid into an absorbent article and also a dry surface and low rewetting are in part incompatible. A close liquid-permeable covering material with fine pores therefore affords good protection against rewetting but leads to the liquid-admission capacity being low. This often results in liquid not being taken up by the absorbent article but instead running out on the surface of the article and causing leakage. Moreover, a fine-pored material tends to retain liquid after wetting, which makes the surface of the article feel wet. A coarse-pored surface material has a good capacity for rapidly admitting liquid and does not to any great extent retain liquid in the pores. On the other hand, such a material offers poor protection against rewetting. A loose material moreover has a low masking effect, which means that coloured bodily fluid such as menstrual blood is clearly visible through the covering material.

In order to remedy the abovementioned problems, it has been proposed to combine different types of liquid-permeable material. EP 0 312 118, for example, describes an absorbent article with a liquid-permeable covering layer arranged over the absorbent body of the article and with a likewise liquid-permeable transport layer arranged between the surface layer and the absorbent body. The transport layer has lower hydrophilicity than the absorbent body and moreover has an effective average pore size which is smaller than the pore size in the surface layer.

US 5,968,855 describes a nonwoven material which is stated to have good liquid-transport properties and can be used as a transport layer in an absorbent article.

In spite of great efforts having been made in order to improve the liquid-permeable covering layer on an absorbent article, it has hitherto not been possible for an optimum combination of covering material and liquid-transfer material to be presented. A main object of the invention is therefore to offer an improved surface material combination, which makes possible both rapid liquid admission and a dry surface with low rewetting.

DISCLOSURE OF INVENTION

In accordance with the invention, an absorbent article of the kind referred to in the introduction has therefore been produced, which article is characterized mainly in that the liquid-permeable covering layer consists of a nonwoven material with a pore volume distribution curve with a maximum at a pore radius greater than or equal to 50 $\mu$m and with a wetting angle of at least 120°, and also in that the liquid-transfer layer consists of a fibrous layer with a pore volume distribution curve with a maximum at a pore radius of from 105 to 325 $\mu$m.

Advantageously, the liquid-permeable covering layer has a pore volume distribution curve with a maximum at a pore radius greater than or equal to 55 $\mu$m and preferably with a maximum at a pore radius of from 555 $\mu$m to 60 $\mu$m.

Furthermore, the liquid-permeable covering layer can consist of fibres with a fibre fineness of at least 5 dtex.

The liquid-permeable covering layer suitably has a basis weight of at most 15 g/m$^2$.

An especially advantageous material for use as a liquid-permeable covering layer has been found to be a relatively hydrophobic spunbond material. Such a material does not spread liquid in the layer, which carded nonwoven materials have a tendency to do.

Furthermore, it is advantageous if the liquid-transfer layer has a pore volume distribution curve with a maximum at a pore radius of from 115 $\mu$m to 185 $\mu$m and preferably with a maximum at a pore radius of from 135 $\mu$m to 155 $\mu$m.

It is furthermore suitable if the liquid-transfer layer has a cumulative pore volume in the pore size range 110 to 350 $\mu$m which is more than 60% of the total pore volume and preferably more than 65% of the total pore volume. In this connection, it is preferred if the liquid-transfer layer has a cumulative pore volume in the pore size range 120 to 230 $\mu$m which is more than 40% of the total pore volume and preferably more than 50% of the total pore volume, and it is most preferable if the liquid-transfer layer has a cumulative pore volume in the pore size range 150 to 180 $\mu$m which is more than 15% of the total pore volume and preferably more than 20% of the total pore volume.

The liquid-transfer layer suitably consists of fibres with a fibre fineness of from 6.7 to 11 dtex.

Furthermore, the liquid-transfer layer advantageously has a basis weight of from 10 gsm to 100 gsm, preferably from 25 gsm to 60 gsm, and a bulk of at least 15 cm$^3$/g measured at a load of 0.1 kPa.

It has also been found to be advantageous if the liquid-transfer layer has a pore volume distribution curve with a maximum located at from 155 $\mu$m to 165 $\mu$m in combination with a cumulative liquid volume of 0.1 mm$^3$/mg of sample and preferably 0.5 mm$^3$/mg or more in pores with radii smaller than or equal to 25 $\mu$m.

The absorbent article according to the invention is, for example, a diaper, an incontinence pad, a sanitary towel, a bed protector or the like and suitably comprises a liquid-impermeable covering layer located over a second surface on the absorbent body opposite the first surface, where the liquid-permeable covering layer and the liquid-impermeable covering layer together enclose the absorbent body.

With regard to both surface dryness and liquid take-up time, it is important, as mentioned above, to use open materials with relatively large pores. It was not previously known, however, which degree of openness gives the best combination of liquid admission and surface dryness.

By virtue of the invention, it is possible to produce absorbent articles with extremely good liquid-handling properties. With knowledge of the nature of a certain material combination, it is also possible to predict how the material combination will behave when it is used on an absorbent article.

As far as dryness is concerned, the properties of both the liquid-permeable covering layer and the liquid-transfer layer are important, although the properties of the liquid-transfer layer have the greatest influence on the dryness. In this connection, the pore volume distribution (PVD) in particular is significant.

DESCRIPTION OF FIGURES

The invention will be described below with reference to the figures shown in the accompanying drawings, in which:

Fig. 1     shows an incontinence pad with a surface material according to the invention;

Fig. 2     shows a section along the line II-II through the incontinence pad in Figure 1;

Fig. 3     shows a drop of liquid arranged on a surface;

Fig. 4     shows a curve chart showing the pore volume distribution for liquid-permeable covering materials;

Fig. 5     shows a curve chart showing the pore volume distribution for liquid-transfer materials;

Fig. 6     shows results of sensory dryness measurements, and

Fig. 7     shows results of sensory dryness measurements.

DETAILED DESCRIPTION OF THE INVENTION

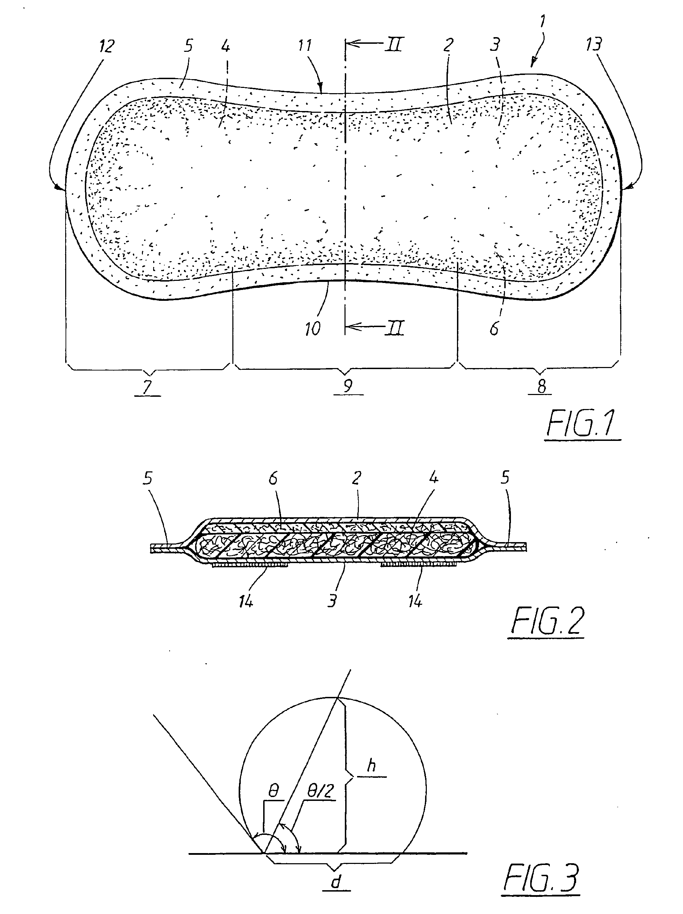

The incontinence pad 1 shown in Figures 1 and 2 comprises a first, liquid-permeable covering layer 2, a second, liquid-impermeable covering layer 3, and an absorbent body 4 enclosed between the covering layers. The two covering layers 2, 3 have a slightly greater extent in the plane than the absorbent body 4 and project beyond the absorbent body 4 around its entire periphery. The covering layers 2, 3 are interconnected within the projecting portions 5, for example by means of gluing or welding using heat or ultrasound.

In accordance with the invention, the liquid-permeable covering layer 2 consists of a layer of nonwoven material. Especially preferred nonwoven materials are spunbond materials.

The liquid-impermeable covering layer 3 can consist of a liquid-impermeable plastic film, a nonwoven layer which has been coated with a liquid-blocking material, or another flexible material layer which is capable of resisting liquid penetration. In general, it is an advantage if the liquid-impermeable covering layer 3 has a certain breathability, that is to say allows the passage of water vapour through the layer 3.

Furthermore, a liquid-permeable liquid-transfer layer 6 is arranged between the liquid-permeable covering layer 3 and the absorbent body 4. Such a liquid-transfer layer 6 consists of a bulky fibrous material with large internal volume. Suitable materials for the liquid-transfer layer 6 are various types of preferably bonded fibre waddings, for example carded, adhesive-bonded or thermally bonded wadding.

The incontinence pad 1 has an elongate shape, with wider end portions 7, 8 and a narrower crotch portion 9. The crotch portion 9 is the part of the incontinence pad 1 which is intended to be arranged in the crotch of the wearer during use and serve as the receiving surface for the bodily fluid which is discharged into the incontinence pad 1. The incontinence pad 1 also has two inwardly curved, longitudinal side edges 10, 11 and two end edges 12, 13.

Arranged on the outside of the liquid-impermeable covering layer 3 is a fastening means 14 in the form of two transverse areas of self-adhesive glue. Before use, the fastening means 14 is suitably covered by a removable protective layer (not shown in the drawing) of release-agent-treated paper, plastic film or the like. Instead of the glue pattern in the form of two transverse glue areas shown, a number of other glue patterns can be used, such as one or more longitudinal areas, dots, full coating etc. Alternatively, other types of fastening means can be used, such as hook and loop surfaces, press studs, belts, special briefs or the like.

An incontinence pad 1 of the kind shown in the figures is primarily intended for use by people with relatively mild incontinence and is therefore of such a size that it can easily be accommodated inside a pair of ordinary briefs. In this connection, the fastening means 14 serves to hold the incontinence pad in place inside the briefs during use.

The absorbent body 4 is shown diagrammatically and consists of absorbent material. The absorbent body 4 can comprise one or more layers which can be the same or differ with regard to composition, shape, size and positioning in the incontinence pad.

Absorption materials which can be used are cellulose fibres, the most common in this respect being cellulose fluff pulp, various types of absorbent foam material, and also what are known as superabsorbents, which are polymer materials which absorb liquid corresponding to many times their own weight while forming a liquid-containing gel. Superabsorbents are available in the form of fibres, particles, granules, film etc. and can be mixed with other absorption materials or be arranged in separate layers or areas.

Although, for the purpose of illustration, the invention has been described here on the basis of an incontinence pad, it is of course the case that other types of absorbent article, such as diapers for children and adults, bed protectors, seat protectors, sanitary towels or the like are also covered by the invention. Absorbent articles such as, for example, diapers can comprise further components which have not been described here and which are not significant for the invention. Examples of such components are elastic means, fastening-together means, wet indicators, raised portions, side barriers etc.

In order to determine the properties of different liquid-permeable covering materials and liquid-transfer layers, and to assess the suitability of different material combinations with regard to dryness and liquid-admission capacity, a number of measurements were performed.

MEASURING METHODS

Determination of wetting angle: DAT (Dynamic Absorption Tester)

In order to determine the degree of hydrophobicity of suitable liquid-permeable covering materials, the following method was used:

A drop of liquid is applied to a test material while a video system films the procedure. Depending on the nature of the test material, the drop may remain lying on top of the material or be absorbed. By measuring the base (d) and the height (h) as shown in Figure 3, the contact angle $\theta$ formed between the liquid and the material can be calculated with the aid of the following equation:

$$\tan\frac{\theta}{2} = \frac{2 \cdot h}{d}$$

The contact angle $\theta$ is stated as a function of the time, t, which passed from the drop coming into contact with the surface of the test material. In the examples below, the contact angle is shown at $t = 0.1$ s, when all the drops were still lying on the surface.

All the test materials were conditioned for at least 4 h before measurement (23°C; 50% relative humidity). The measurements were carried out on a Fibro 1100 DAT system from Fibro System AB, Sweden, in accordance with the associated manual (software version: DAT WinNT 3.0). Water was used as the test liquid and the drop volume was 5 $\mu$l. 25 drops were measured for each material.

The materials for which the contact angle was determined were:

N1, which was a spunbond nonwoven with a weight per unit area of 17.9 g/m$^2$ and a fibre fineness of 3.2 dtex.
N2, which was a spunbond nonwoven with a weight per unit area of 14.9 g/m$^2$ and a fibre fineness of 3.8 dtex.

N3, which was a spunbond nonwoven with a weight per unit area of 15.9 g/m$^2$ and a fibre fineness of 3.7 dtex.
N4, which was a spunbond nonwoven with a weight per unit area of 11.3 g/m$^2$ and a fibre fineness of 4.9 dtex.
N5, which was a spunbond nonwoven with a weight per unit area of 15 g/m$^2$ and a fibre fineness of 5 dtex.

N7, which was a spunbond nonwoven with a weight per unit area of 13.3 g/m$^2$ and a fibre fineness of 5.4 dtex.
N8, which was a spunbond nonwoven with a weight per unit area of 15 g/m$^2$ and a fibre fineness of 5.8 dtex.
N9, which was a spunbond nonwoven with a weight per unit area of 13.2 g/m$^2$ and a fibre fineness of 7.1 dtex.
N10, which was a spunbond nonwoven with a weight per unit area of 22 g/m$^2$ and a fibre fineness of 2.3 dtex.
N11, which was a spunbond nonwoven with a weight per unit area of 16 g/m$^2$ and a fibre fineness of 3.6 dtex.

The result of the measurements is indicated in Table 1.

Table 1: Wetting angles

| Material | Contact angle (t = 0.1 s) | Number of drops measured |
|---|---|---|
| N1 | 125 | 25 |
| N2 | 124 | 25 |
| N3 | 116 | 25 |
| N4 | 123 | 25 |
| N5 | 123 | 25 |
| N6 | 121 | 25 |
| N7 | 114 | 25 |
| N8 | 120 | 25 |
| N9 | 123 | 25 |
| N10 | 0 | 25 |
| N11 | 122 | 25 |

DETERMINATION OF PORE VOLUME DISTRIBUTION

The pore volume distribution for different liquid-permeable covering materials and liquid-transfer materials was determined using the method described in Journal of Colloid and Interface Science 162, 163-170 (1994). The method used is based on measurements of the quantity of liquid which can be pressed out of a porous material ("receding mode") at a certain pressure, and the result of the measurement is presented in the form of a curve in a chart where the curve illustrates the overall pore volume for a given pore radius.

Running conditions for liquid-permeable covering layer (nonwoven)

In the measurements, n-hexadecane (≥99%, Sigma H-0255) was used as the measuring liquid. Measurement was carried out on circular samples with an area of 25.5 cm$^2$. The sample was placed in the chamber and was saturated with the test liquid. Millipore 0.22 μm cat. no. GSWP 09000 was used as the membrane. In order to achieve good contact between the sample and the membrane, a load covering the whole sample surface was placed on top of the sample. In order to avoid measuring pores between the sample surface and the weight, a large-pore polyurethane foam (which does not hold liquid) was moreover placed between the sample and the weight applied. The total load on the sample was 0.15 kPa. In order for it to be possible to record the remaining liquid, the sample was weighed before and immediately after running was completed.
The equilibrium speed, that is to say the speed when the weight change at the selected pore radius has decreased to an insignificant level, was set at 2 mg/min, and the measuring time during which the weight change was recorded was set at 30 seconds.
Measurements were carried out at pressures corresponding to the following pore radii [μm]:

| 500 | 400 | 300 | 250 | 225 | 200 |
|---|---|---|---|---|---|
| 175 | 150 | 125 | 110 | 100 | 90 |

(continued)

| | | | | | |
|---|---|---|---|---|---|
| 80 | 70 | 60 | 55 | 50 | 45 |
| 40 | 35 | 40 | 25 | 20 | 15 |
| 10 | 8 | 6 | 4 | 2 | |

In addition to measurement on samples, what is known as a blank run was carried out. In a blank run, only foam and load are placed in the test chamber. Measurement is performed in the same way and with the same running conditions as when samples are measured. The blank run is then subtracted from the test run before further processing of raw data.

Running conditions for liquid-transfer layer (wadding)

In the measurements, a 0.1% w/w solution of Triton TX-100 (Calbiochem - 648462) was used as the measuring liquid. Measurement was carried out on circular samples with an area of 25.5 cm$^2$. The sample was placed in the chamber and was saturated with the test liquid. Millipore 0.22 $\mu$m cat. no. GSWP 09000 was used as the membrane. In order to achieve good contact between the sample and the membrane, a load covering the whole sample surface was placed on top of the sample. In order to avoid measuring pores between the sample surface and the weight, a large-pore polyurethane foam (which does not hold liquid) was moreover placed between the sample and the weight applied. The total load on the sample was 0.57 kPa.

The equilibrium speed, that is to say the speed when the weight change at the selected pore radius has decreased to an insignificant level, was set at 5 mg/min, and the measuring time during which the weight change was recorded was set at 30 seconds.

Measurements were carried out at pressures corresponding to the following pore radii [$\mu$m]:

| | | | | | |
|---|---|---|---|---|---|
| 700 | 600 | 500 | 400 | 350 | 300 |
| 275 | 250 | 240 | 230 | 220 | 210 |
| 200 | 190 | 180 | 170 | 160 | 150 |
| 140 | 130 | 120 | 110 | 100 | 90 |
| 80 | 70 | 60 | 50 | 40 | 30 |
| 25 | 20 | 15 | 10 | 5 | |

In addition to measurement on samples, a blank run was carried out. In a blank run, only foam and load are placed in the test chamber. Measurement is performed in the same way and with the same running conditions as when samples are measured. The blank run is then subtracted from the test run before further processing of raw data.

The wadding materials for which the pore volume distribution was determined were:

V1, which was an adhesive-bonded polyester wadding with a weight per unit area of 43.0 g/m$^2$ and a fibre fineness of 5.3 dtex.

V2, which was an adhesive-bonded polyester wadding with a weight per unit area of 34.1 g/m$^2$ and a fibre fineness of 6.7 dtex.

V3, which was an adhesive-bonded polyester wadding with a weight per unit area of 50.4 g/m$^2$ and a fibre fineness of 6.7 dtex.

V4, which was an adhesive-bonded polyester wadding with a weight per unit area of 28.3 g/m$^2$ and a fibre fineness of 7.7 dtex.

V5, which was an adhesive-bonded polyester wadding with a weight per unit area of 38.0 g/m$^2$ and a fibre fineness of 7.7 dtex.

V6, which was an adhesive-bonded polyester wadding with a weight per unit area of 59.9 g/m$^2$ and a fibre fineness of 7.7 dtex.

V8, which was an adhesive-bonded polyester wadding with a weight per unit area of 50.7 g/m$^2$ and a fibre fineness of 8.8 dtex.

V9, which was an adhesive-bonded polyester wadding with a weight per unit area of 61.7 g/m$^2$ and a fibre fineness of 8.8 dtex.

V10, which was an adhesive-bonded polyester wadding with a weight per unit area of 50.0 g/m$^2$ and a fibre fineness of 6.7 dtex.

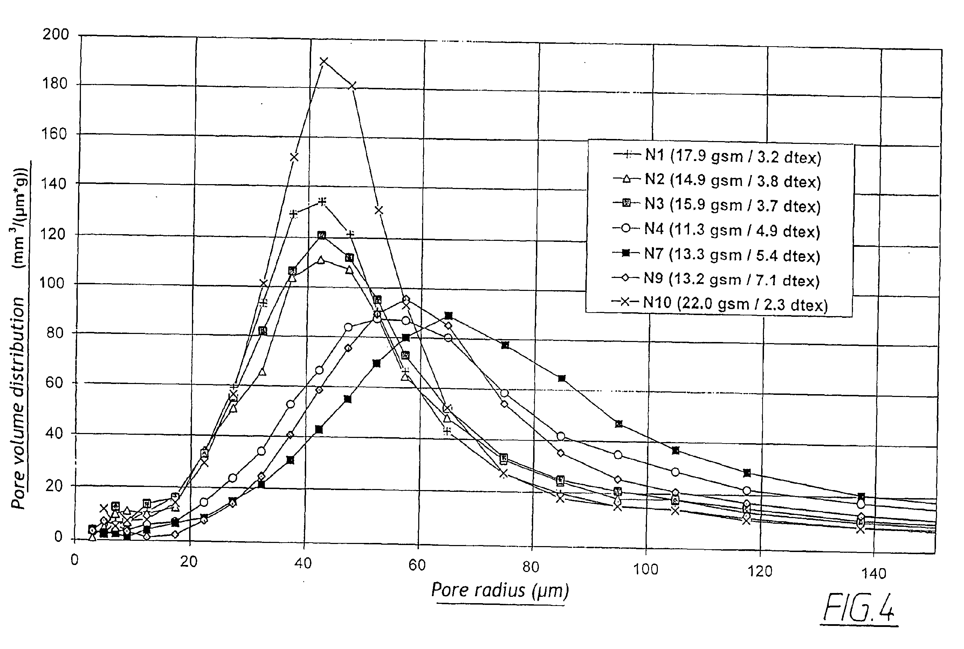

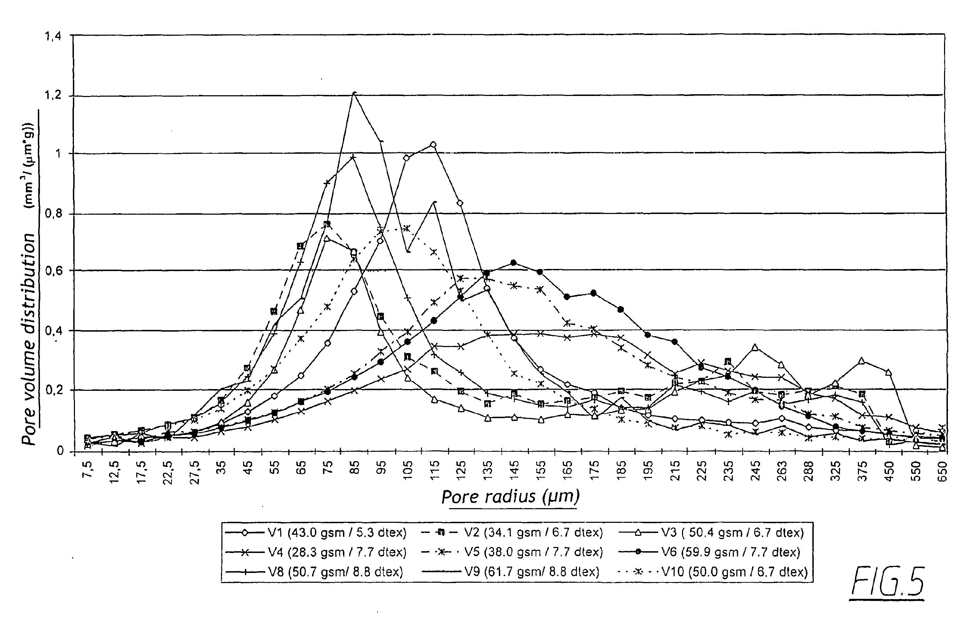

The results of the measurements are illustrated in the form of pore volume distribution curves in Figures 4 and 5.

Figure 4 shows the pore volume distribution for different liquid-permeable covering materials. The samples on which measurements were performed were N1-N4, N7 and N9-N10, which were described above in connection with determination of hydrophobicity/contact angle.

The best pore volume distribution is that demonstrated by N9, but N4 is also very good, and the other tested materials show an acceptable pore volume distribution.

Figure 5 illustrates the results of the measurements on different liquid-transfer materials, V1-V9, and also a reference, V10.

Table 2 below shows the liquid percentage within the pore radius ranges 110-350 $\mu$m, 120-230 $\mu$m and 150-180 $\mu$m of the total cumulative volume in the range 0-700 $\mu$m for the samples V1-V10:

Table 2: Liquid percentage for different pore radius ranges

| Pore radius range: | 110-350 $\mu$m | 120-230 $\mu$m | 150-180 $\mu$m |
|---|---|---|---|
| V1 | 57.9 | 39.3 | 10.4 |
| V2 | 49.1 | 23.2 | 6.6 |
| V3 | 51.6 | 17.9 | 4.4 |
| V4 | 64.4 | 41.5 | 15.5 |
| V5 | 64.0 | 47.2 | 18.6 |
| V6 | 66.5 | 52.1 | 21.3 |
| V8 | 46.3 | 21.7 | 6.0 |
| V9 | 43.3 | 31.7 | 8.8 |
| V10 | 39.8 | 27.0 | 7.0 |

The liquid percentage within the ranges concerned provides a measure of how well defined a curve peak located within the range is.

Dryness determination

The perceived dryness of the different material combinations was determined by sensory evaluation.

The test liquid was synthetic urine, SUM, made up as follows: 0.66 g/l magnesium sulphate; 4.47 g/l potassium chloride, 7.60 g/l sodium chloride, 18.00 g/l urea, 3.54 g/l potassium dihydrogen phosphate, 0.745 g/l sodium hydrogen phosphate, 1.00 g/l 0.1 % triton, 0.4 g/l Nykockin (colour) and the rest de-ionized water. The test liquid was applied to the samples in 3 doses of 100 ml, with intervals of 20 minutes between doses. The dryness evaluation was carried out 20 minutes after the last dose was added.

Different sensory methods were used in order to characterize the degree of dryness of the samples concerned:

Method 1: A panel consisting of people well informed of product type, evaluation procedure and attribute asked for has to place in order up to 10 samples on each occasion. All the samples are evaluated in a number of different combinations with other samples. The samples are evaluated blind. The ordering results are put together, and the samples are grouped so that each group consists of samples with similar dryness. For detailed information about the test procedure, see Sensory Evaluation Techniques, 2nd edition, ISBN 0-8493-4280-5. Authors: Meilgaard, Cicille & Carr. Pages 117-119 are of particular interest.

Method 2: The samples within one and the same group are arranged in random order and evaluated by two expert evaluators. The expert evaluators are well informed of product type, evaluation procedure and attribute asked for. Samples within the same group are placed in order according to increasing degree of dryness. New groups are then formed from two similar groups in such a way that the driest samples from a wetter group and the wetter samples from a drier group are brought together, presented in random order and placed in order according to increasing degree of dryness. When all the original and newly formed groups have been placed in order in increasing degree of dryness, all the samples included are evaluated in the order produced. In the tests reported below, the samples were evaluated blind on all occasions.

As the samples consist of mutually different materials and material combinations, where the materials in themselves vary slightly, depending on where the sample is taken from, all evaluations were carried out several times.

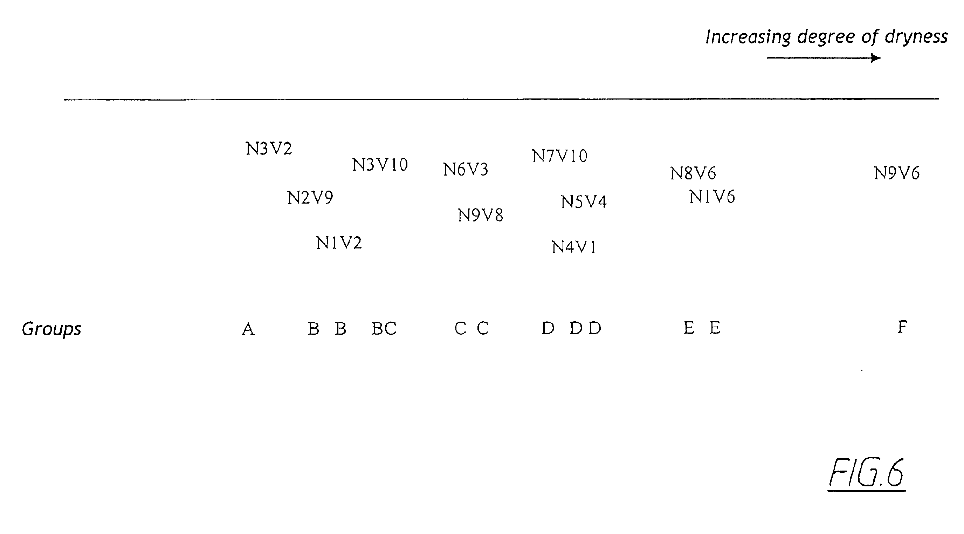

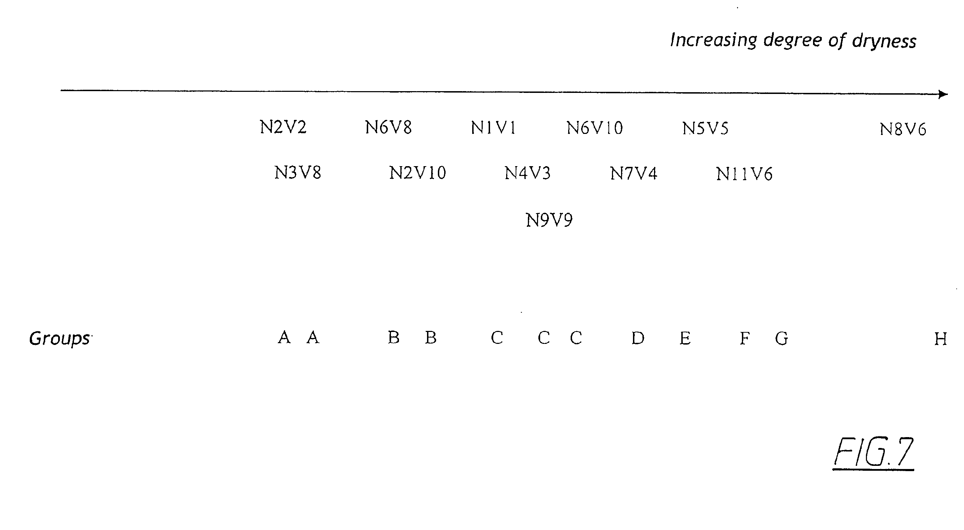

Results of dryness evaluations, performed according to method 2 above, for different combinations of surface material (NX) and underlying wadding (VX) are shown in Figures 6 and 7.

LIQUID-ADMISSION SPEED

The capacity for rapidly taking up liquid and allowing it to pass through was measured for different material combinations. The measurements were carried out in accordance with the ART method which is described in detail in British patent specification GB 2 339 477.

The ART method is based on measuring the time it takes for an absorbent structure to receive a given quantity of liquid from a vessel, the quantity of liquid being measured continuously by measuring the quantity of liquid which is carried away from the vessel.

The results for the different material combinations tested are presented below in Table 3, which shows a comparison on a percentage basis of the admission times which were required to carry away the added quantity of liquid (SUM, made up as above) for different combinations of surface material and liquid-transfer layer, where the admission time for Sample 5 is set at 100 for doses 1, 2 and 3. The test liquid was added at 100 ml/dose with an 8 ml liquid column and at a pressure of 4.5 kg.

Table 3: Liquid-admission time

|  | N9+V6 | N9+V2 | N3+V6 | N3+V4 | N10+V10 | N9+V1 |
|---|---|---|---|---|---|---|
| ART | Sample 1 | Sample 2 | Sample 3 | Sample 4 | Sample 5 | Sample 6 |
| 1st dose | 96 | 112 | 92 | 197 | 100 | 84 |
| 2nd dose | 100 | 123 | 99 | 121 | 100 | 80 |
| 3rd dose | 96 | 123 | 109 | 126 | 100 | 93 |

The invention is not to be considered as being limited to the illustrative embodiments described here. The invention therefore comprises all types of absorbent article intended for absorption of bodily fluids such as urine, loose motions and blood.

**Claims**

1. An absorbent article (1) comprising an absorbent body (4), a liquid-permeable covering layer.(2) arranged over a first surface on the absorbent body (4), and a liquid-permeable liquid-transfer layer (6) arranged between the absorbent body (4) and the liquid-permeable covering layer (2), **characterized in that** the liquid-permeable covering layer (2) consists of a nonwoven material with a pore volume distribution curve with a maximum at a pore radius greater than or equal to 50 $\mu$m and with a wetting angle of at least 120°, and also **in that** the liquid-transfer layer (6) consists of a fibrous layer with a pore volume distribution curve with a maximum at a pore radius of from 105 to 325 $\mu$m and **in that** the liquid-transfer layer (6) has a basis weight of from 10 gsm to 100 gsm, preferably from 25 gsm to 60 gsm, and a bulk of at least 15 cm$^3$/g measured at a load of 0.1 kPa.

2. An absorbent article according to Claim 1, **characterized in that** the liquid-permeable, covering layer (2) has a pore volume distribution curve with a maximum at a pore radius greater than or equal to 55 $\mu$m.

3. An absorbent article according to Claim 2, **characterized in that** the liquid-permeable covering layer (2) has a pore volume distribution curve with a maximum at a pore radius of from 55 $\mu$m to 60 $\mu$m.

4. An absorbent article according to any one of the preceding claims, **characterized in that** the liquid-permeable covering layer (2) consists of fibres with a fibre fineness of at least 5 dtex.

5. An absorbent article according to any one of the preceding claims, **characterized in that** the liquid-permeable covering layer (2) has a basis weight of at most 15 g/m$^2$.

6. An absorbent article according to any one of the preceding claims, **characterized in that** the liquid-permeable covering layer (2) consists of a spunbond nonwoven.

7. An absorbent article according to any one of the preceding claims, **characterized in that** the liquid-transfer layer (6) consists of a polyester wadding bonded with a binding agent.

8. An absorbent article according to any one of the preceding claims, **characterized in that** the liquid-transfer layer (6) has a pore volume distribution curve with a maximum at a pore radius of from 115 $\mu$m to 185 $\mu$m.

9. An absorbent article according to Claim 8, **characterized in that** the liquid-transfer layer (6) has a pore volume distribution curve with a maximum at a pore radius of from 135 $\mu$m to 155 $\mu$m.

10. An absorbent article according to any one of the preceding claims, **characterized in that** the liquid-transfer layer (6) has a cumulative pore volume in the pore size range 110 to 350 $\mu$m which is more than 60% of the total pore volume and preferably more than 65% of the total pore volume.

11. An absorbent article according to Claim 10, **characterized in that** the liquid-transfer layer (6) has a cumulative pore volume in the pore size range 120 to 230 $\mu$m which is more than 40% of the total pore volume and preferably more than 50% of the total pore volume.

12. An absorbent article according to Claim 11, **characterized in that** the liquid-transfer layer (6) has a cumulative pore volume in the pore size range 150 to 180 $\mu$m which is more than 15% of the total pore volume and preferably more than 20% of the total pore volume.

13. An absorbent article according to any one of the preceding claims, **characterized in that** the liquid-transfer layer (6) consists of fibres with a fibre fineness of from 8.7 to 11 dtex.

14. An absorbent article according to any one of the preceding claims, **characterized in that** the article comprises a liquid-impermeable covering layer (3) located over a second surface on the absorbent body (4) opposite the first surface, and **in that** the liquid-permeable covering layer (2) and the liquid-impermeable covering layer (3) together enclose the absorbent body (4).

**Patentansprüche**

1. Saugfähiger Artikel (1), umfassend einen saugfähigen Körper (4), eine flüssigkeitsdurchlässige Deckschicht (2), die über einer ersten Oberfläche auf dem saugfähigen Körper (4) angeordnet ist, und eine flüssigkeitsdurchlässige Flüssigkeitsüberführungsschicht (6), die zwischen dem saugfähigen Körper (4) und der flüssigkeitsdurchlässigen Deckschicht (2) angeordnet ist, **dadurch gekennzeichnet, dass** die flüssigkeitsdurchlässige Deckschicht (2) aus einem Vliesmaterial mit einer Porenvolumenverteilungskurve mit einem Maximum bei einem Porenradius größer als oder gleich 50 $\mu$m und mit einem Benetzungswinkel von mindestens 120° besteht, und auch dass die Flüssigkeitsüberführungsschicht (6) aus einer Faserschicht mit einer Porenvolumenverteilungskurve mit einem Maximum bei einem Porenradius von 105 bis 325 $\mu$m besteht, und dass die Flüssigkeitsüberführungsschicht (6) ein Basisgewicht von 10 gsm bis 100 gsm, vorzugsweise von 25 gsm bis 60 gsm, und eine Masse von mindestens 15 cm$^3$/g gemessen bei einer Belastung von 0,1 kPa aufweist.

2. Saugfähiger Artikel nach Anspruch 1, **dadurch gekennzeichnet, dass** die flüssigkeitsdurchlässige Deckschicht (2) eine Porenvolumenverteilungskurve mit einem Maximum bei einem Porenradius größer als oder gleich 55 $\mu$m aufweist.

3. Saugfähiger Artikel nach Anspruch 2, **dadurch gekennzeichnet, dass** die flüssigkeitsdurchlässige Deckschicht (2) eine Porenvolumenverteilungskurve mit einem Maximum bei einem Porenradius von 55 $\mu$m bis 60 $\mu$m.

4. Saugfähiger Artikel nach irgendeinem der vorhergehenden Ansprüche, **dadurch gekennzeichnet, dass** die flüssigkeitsdurchlässige Deckschicht (2) aus Fasern mit einer Faserfeinheit von mindestens 5 dtex besteht.

5. Saugfähiger Artikel nach irgendeinem der vorhergehenden Ansprüche, **dadurch gekennzeichnet, dass** die flüssigkeitsdurchlässige Deckschicht (2) ein Basisgewicht von höchstens 15 g/m$^2$ aufweist.

6. Saugfähiger Artikel nach irgendeinem der vorhergehenden Ansprüche, **dadurch gekennzeichnet, dass** die flüssigkeitsdurchlässige Deckschicht (2) aus einem Spinnvlies besteht.

**7.** Saugfähiger Artikel nach irgendeinem der vorhergehenden Ansprüche, **dadurch gekennzeichnet, dass** die Flüssigkeitsüberführungsschicht (6) aus einem mit einem Bindemittel gebundenen Polyesterfüllmaterial besteht.

**8.** Saugfähiger Artikel nach irgendeinem der vorhergehenden Ansprüche, **dadurch gekennzeichnet, dass** die Flüssigkeitsüberführungsschicht (6) eine Porenvolumenverteilungskurve mit einem Maximum bei einem Porenradius von 115 μm bis 185 μm aufweist.

**9.** Saugfähiger Artikel nach Anspruch 8, **dadurch gekennzeichnet, dass** die Flüssigkeitsüberführungsschicht (6) eine Porenvolumenverteilungskurve mit einem Maximum bei einem Porenradius von 135 μm bis 155 μm aufweist.

**10.** Saugfähiger Artikel nach irgendeinem der vorhergehenden Ansprüche, **dadurch gekennzeichnet, dass** die Flüssigkeitsüberführungsschicht (6) ein kumulatives Porenvolumen im Porengrößenbereich von 110 bis 350 μm aufweist, was mehr als 60 % des gesamten Porenvolumens und vorzugsweise mehr als 65 % des gesamten Porenvolumens ist.

**11.** Saugfähiger Artikel nach Anspruch 10, **dadurch gekennzeichnet, dass** die Flüssigkeitsüberführungsschicht (6) ein kumulatives Porenvolumen im Porengrößenbereich von 120 bis 230 μm aufweist, was mehr als 40 % des gesamten Porenvolumens und vorzugsweise mehr als 50 % des gesamten Porenvolumens ist.

**12.** Saugfähiger Artikel nach Anspruch 11, **dadurch gekennzeichnet, dass** die Flüssigkeitsüberführungsschicht (6) ein kumulatives Porenvolumen im Porengrößenbereich von 150 bis 180 μm aufweist, was mehr als 15 % des gesamten Porenvolumens und vorzugsweise mehr als 20 % des gesamten Porenvolumens ist.

**13.** Saugfähiger Artikel nach irgendeinem der vorhergehenden Ansprüche, **dadurch gekennzeichnet, dass** die Flüssigkeitsüberführungsschicht (6) aus Fasern mit einer Faserfeinheit von 6,7 bis 11 dtex besteht.

**14.** Saugfähiger Artikel nach irgendeinem der vorhergehenden Ansprüche, **dadurch gekennzeichnet, dass** der Artikel eine flüssigkeitsundurchlässige Deckschicht (3) umfasst, die über einer zweiten Oberfläche auf dem saugfähigen Körper (4) gegenüber der ersten Oberfläche angeordnet ist, und dass die flüssigkeitsdurchlässige Deckschicht (2) und die flüssigkeitsundurchlässige Deckschicht (3) zusammen den saugfähigen Körper (4) einschließen.


**Revendications**

**1.** Article absorbant (1) comprenant un corps absorbant (4), une couche de revêtement perméable aux liquides (2) disposée au-dessus d'une première surface sur le corps absorbant (4), et une couche de transfert de liquide perméable aux liquides (6) disposée entre le corps absorbant (4) et la couche de revêtement perméable aux liquides (2), **caractérisé en ce que** la couche de revêtement perméable aux liquides (2) est constituée d'une matière non tissée présentant une courbe de distribution du volume poreux avec un maximum au niveau du rayon poreux supérieur à ou égal à 50 μm et dont l'angle de mouillage est d'au moins 120°, et également **en ce que** la couche de transfert de liquide (6) est constituée d'une couche fibreuse présentant une courbe de distribution du volume poreux avec un maximum au niveau du rayon poreux compris entre 105 et 325 μm et **en ce que** la couche de transfert de liquide (6) présente un poids de base compris entre 10 gsm et 100 gsm, préférablement entre 25 gsm et 60 gsm, et une masse surfacique d'au moins 15 cm$^3$/g mesurée à une charge de 0,1 kPa.

**2.** Article absorbant selon la revendication 1, **caractérisé en ce que** la couche de revêtement perméable aux liquides (2) présente une courbe de distribution de volume des pores avec un maximum à un rayon de pores supérieur à ou égal à 55 μm.

**3.** Article absorbant selon la revendication 2, **caractérisé en ce que** la couche de revêtement perméable aux liquides (2) présente une courbe de distribution de volume des pores avec un maximum à un rayon de pores compris entre 55 μm et 60 μm.

**4.** Article absorbant selon l'une quelconque des revendications précédentes, **caractérisé en ce que** la couche de revêtement perméable aux liquides (2) est constituée de fibres ayant une finesse de fibre d'au moins 5 dtex.

**5.** Article absorbant selon l'une quelconque des revendications précédentes, **caractérisé en ce que** la couche de revêtement perméable aux liquides (2) présente un poids de base d'au plus 15 g/m$^2$.

**6.** Article absorbant selon l'une quelconque des revendications précédentes, **caractérisé en ce que** la couche de revêtement perméable aux liquides (2) est constituée d'un non-tissé filé-lié.

**7.** Article absorbant selon l'une quelconque des revendications précédentes, **caractérisé en ce que** la couche de transfert de liquide (6) est constituée d'une ouate de polyester aggloméré avec un liant.

**8.** Article absorbant selon l'une quelconque des revendications précédentes, **caractérisé en ce que** la couche de transfert de liquide (6) présente une courbe de distribution de volume des pores avec un maximum à un rayon de pores compris entre 115 $\mu$m et 185 $\mu$m.

**9.** Article absorbant selon la revendication 8, **caractérisé en ce que** la couche de transfert de liquide (6) présente une courbe de distribution de volume des pores avec un maximum à un rayon de pores compris entre 135 $\mu$m et 155 $\mu$m.

**10.** Article absorbant selon l'une quelconque des revendications précédentes, **caractérisé en ce que** la couche de transfert de liquide (6) présente un volume de pores cumulé dans la gamme de taille de pores comprise entre 110 et 350 $\mu$m ce qui est plus de 60% du volume poreux total et de préférence plus de 65% du volume poreux total.

**11.** Article absorbant selon la revendication 10, **caractérisé en ce que** la couche de transfert de liquide (6) présente un volume de pores cumulé dans la gamme de taille de pores comprise entre 120 et 230 $\mu$m ce qui est plus de 40% du volume poreux total et de préférence plus de 50% du volume poreux total.

**12.** Article absorbant selon la revendication 11, **caractérisé en ce que** la couche de transfert de liquide (6) présente un volume de pores cumulé dans la gamme de taille de pores comprise entre 150 et 180 $\mu$m ce qui est plus de 15% du volume poreux total et de préférence plus de 20% du volume poreux total.

**13.** Article absorbant selon l'une quelconque des revendications précédentes, **caractérisé en ce que** la couche de transfert de liquide (6) est constituée de fibres ayant une finesse de fibre comprise entre 6,7 et 11 dtex.

**14.** Article absorbant selon l'une quelconque des revendications précédentes, **caractérisé en ce que** l'article comprend une couche de revêtement imperméable aux liquides (3) située au-dessus d'une deuxième surface sur le corps absorbant (4) opposé à la première surface, et **en ce que** la couche de revêtement perméable aux liquides (2) et la couche de revêtement imperméable aux liquides (3) ensemble entourent le corps absorbant (4).

_FIG.1_

_FIG.2_

_FIG.3_

FIG.4

FIG.5

*Increasing degree of dryness*

→

N3V2

       N3V10   N6V3    N7V10

                                      N8V6        N9V6

N2V9                       N5V4

                   N9V8                      N1V6

N1V2                       N4V1

*Groups*     A   B  B  BC    C  C    D  D D    E  E        F

*FIG.6*

EP 1 603 506 B1

EP 1 603 506 B1

*Increasing degree of dryness*

N2V2        N6V8        N1V1        N6V10        N5V5                    N8V6

N3V8        N2V10        N4V3        N7V4        N11V6

N9V9

*Groups*        A  A        B  B        C        C  C        D        E        F  G                    H

*FIG.7*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0312118 A
- US 5968855 A

- GB 2339477 A

### Non-patent literature cited in the description

- *Journal of Colloid and Interface Science,* 1994, vol. 162, 163-170

- **MEILGAARD ; CICILLE ; CARR.** Sensory Evaluation Techniques. 117-119